Europäisches Patentamt

**European Patent Office**

Office européen des brevets

(11) Numéro de publication: **0 320 366**
A1

# DEMANDE DE BREVET EUROPEEN

(21) Numéro de dépôt: **88403085.9**

(22) Date de dépôt: **06.12.88**

(51) Int. Cl.4: **C 12 P 21/00**
C 07 K 7/10, C 07 K 7/06,
G 01 N 33/573, G 01 N 33/577
//(C12P21/00,C12R1:91)

(30) Priorité: **07.12.87 FR 8716962**

(43) Date de publication de la demande:
**14.06.89 Bulletin 89/24**

(84) Etats contractants désignés: **DE GB**

(71) Demandeur: **COMMISSARIAT A L'ENERGIE ATOMIQUE**
**31/33, rue de la Fédération**
**F-75015 Paris (FR)**

(72) Inventeur: **Menez, André**
**109 avenue Claude Nicolas Ledoux Magny les Hameaux**
**F-78470 Saint Remy Les Chevreuses (FR)**

**Mollier, Pascale**
**4 rue Scipion**
**F-75005 Paris (FR)**

(74) Mandataire: **Ores, Irène et al**
**CABINET ORES 6, Avenue de Messine**
**F-75008 Paris (FR)**

Le titre de l'invention a été modifié (Directives relatives à l'examen pratiqué à l'OEB, A-III, 7.3)

(54) **Anticorps monoclonaux antiphospholipase A2 de venins de serpents des familles des élapidés et des hydrophidés, leurs procédés de préparation et leur utilisation pour le dépistage d'empoisonnement par ces familles de serpents.**

(57) Anticorps monoclonaux antiphospholipase $A_2$ de venins de serpents des familles des Elapidés et des Hydrophidés, leurs procédés de préparation et leur utilisation pour le dépistage antivenimeux.

Les anticorps antiphospholipase $A_2$ (anti-$PLA_2$) de venins de serpents, selon l'invention sont constitués par des anticorps monoclonaux polyspécifiques, c'est-à-dire capables de reconnaître non seulement la $PLA_2$ utilisée pour les préparer, mais également au moins une autre $PLA_2$ toxique de venins de serpents des familles des Elapidés et des Hydrophidés, sont de type $IgG_1K$, présentent une grande affinité pour la notexine, exprimée par leur constante de dissociation, $K_D$, comprise entre $1,4 \times 10^{-9}M$ et $15,8 \times 10^{-9}M$.

Fragments antigéniques d'une pluralité de $PLA_2$ de venins de serpents des familles des Elapidés et des Hydrophidés reconnus par lesdits anticorps monoclonaux polyspécifiques.

Application au dépistage antivenimeux.

EP 0 320 366 A1

Bundesdruckerei Berlin

Description

## ANTICORPS MONOCLONAUX ANTIPHOSPHOLIPASE $A_2$ DE VENINS DE SERPENTS DES FAMILLES DES ELAPIDES ET DES HYDROPHIDES, LEURS PROCEDES DE PREPARATION ET LEUR UTILISATION POUR LE DEPISTAGE ANTIVENIMEUX.

La présente invention est relative à des anticorps monoclonaux antiphospholipase $A_2$ de venins de serpents des familles des Elapidés et des Hydrophidés, à leurs procédés de préparation et à leur utilisation pour le dépistage antivenimeux.

Les venins de serpents renferment de nombreuses protéines dont certaines sont dotées d'une activité enzymatique. C'est le cas des phospholipases $A_2$ qui sont des enzymes capables d'hydrolyser les phospholipides membranaires. On peut classer lesdites phospholipases $A_2$ en trois groupes essentiels. Le premier renferme des phospholipases non toxiques et dont le rôle est avant tout de participer à la digestion des tissus des proies des serpents. Le second groupe renferme des phospholipases $A_2$ hautement toxiques. Ces dernières sont capables de bloquer la libération d'acétylcholine à partir des terminaisons nerveuses. Elles agissent donc en bloquant la transmission neuromusculaire. Ce sont des phospholipases paralysantes qui immobilisent rapidement les proies des serpents. Le troisième groupe comporte des phospholipases moins toxiques que les précédentes. Elles se caractérisent par diverses activités telles que des actions anticoagulantes (VERHEIJ H.M. et al., Eur. J. BIOCHEM, 1980, 112, 25-32), hématotoxiques (ZWAAL R.F.A. et al., BBA, 1975, 406, 83-96), et cytotoxiques (Demande de Brevet français n° 87 16096 du 20 novembre 1987).

Comme on le sait, les $PLA_2$ de venins de serpent sont des protéines qui ont la particularité de présenter une structure hélicoïdale.

Certaines $PLA_2$ ont plusieurs des activités précitées, ce qui rend difficile leur classification. Les $PLA_2$ varient également entre elles par leur degré de toxicité, exprimée par la dose létale 50 % ($DL_{50}$ : quantité de toxine provoquant statistiquement la mort de la moitié de la population des animaux injectés). Les $PLA_2$ peuvent être monomères ou polymères, acides ou basiques (CHANG C.C., Proc. Nat. Sci. Counc., 1985 9-2, 126-142).

Contrairement aux $PLA_2$ neutres ou acides de venins, les $PLA_2$ basiques sont très toxiques, parfois même davantage que les neurotoxines post-synaptiques non phospholipasiques.

La $PLA_2$ monomère la plus toxique est la notexine, extraite du venin de Notechis scutatus scutatus, serpent tigre australien. La toxicité de ce venin est d'ailleurs due essentiellement à cette notexine, responsable de nombreux cas d'envenimation en Australie.

Les $PLA_2$ très toxiques étant létales, il est apparu important de pouvoir déterminer rapidement un empoisonnement par venin chez un patient, même si les symptômes tardent à apparaître.

On sait préparer des anticorps polyclonaux contre les phospholipases appartenant à chaque groupe (CARATSCH C.G. et al., Proc. Royal Soc. Lond.,1982, B 215, 365-373 ; MEIJER H. et al., J. Biol. Chem., 1978, 253, 23, 8564 - 8569). Ces anticorps, de manière très générale, sont capables de neutraliser l'activité létale de la phospholipase $A_2$ contre laquelle ils sont dirigés. En d'autres termes, ces anticorps sont hautement spécifiques. Récemment, une revue détaillée consacrée aux aspects d'immunologie moléculaire des toxines de serpents, a montré l'aspect très aigü de cette spécificité antigénique (MENEZ A., Pharm. Ther., 1985, 30, 91 - 113). Ceci, en pratique, rejoint les phénomènes observés lors de la lutte immunologique contre certains virus, notamment celui de la grippe, à savoir qu'il existe, au sein d'une classe d'éléments nocifs à caractère protéique, une très forte variabilité antigénique, due à des mutations. Lorsque celles-ci se produisent, les zones antigéniques changent de propriétés chimiques et structurales et de ce fait deviennent méconnaissables pour les anticorps précédemment produits. Il faut, pour qu'elles soient à nouveau reconnues, produire d'autres anticorps complémentaires de ces nouvelles zones mutées. C'est en raison du principe de la variabilité antigénique que chaque année, on subit la vaccination contre les nouveaux virus de la grippe.

Il serait donc éminemment souhaitable d'être en mesure de pourvoir à un anticorps dirigé plus spécialement contre des zones protéiques non soumises à de telles mutations. De telles zones existent. Ce sont celles qui assurent, pour une famille d'agents homologues, la communauté de leur action. Des anticorps dirigés contre de telles régions conservées auraient deux avantages : ils permettraient de reconnaître tout agent de la famille considérée ; les anticorps seraient à l'évidence des outils de dépistage de première importance et/ou ils permettraient de neutraliser tout agent de cette famille, indépendamment des mutations subies. Les zones conservées des protéines, cependant, ne sont pas dominantes d'un point de vue immunologique. Les régions soumises à mutations, plus exposées, sont les zones immunologiques dominantes. Ceci ne signifie pas pour autant que les zones conservées n'induisent aucune réponse immunitaire.

La spécificité de reconnaissance des anticorps polyclonaux ne permet pas une détection rapide des $PLA_2$ toxiques, notamment dans un contexte d'urgence médicale.

La présente invention s'est en conséquence donné pour but de pourvoir à des anticorps monoclonaux polyspécifiques, propres à permettre la détection de la présence de venins de serpents des familles des Elapidés et des Hydrophidés dans des fluides biologiques ou l'identification de serpents par leurs venins.

C'est aussi un but de l'invention de pourvoir à des fractions antigéniques correspondant au site antigénique des $PLA_2$ de venin de serpents des familles des Elapidés et des Hydrophidés.

C'est également un but de l'invention de pourvoir à un procédé de détection d'empoisonnement par un venin de serpent des familles des Elapidés et des Hydrophidés dans un fluide biologique.

C'est en outre un but de l'invention de pourvoir à un kit de dépistage antivenimeux, concernant les serpents des familles des Elapidés et des Hydrophidés.

La présente invention a pour objet des anticorps anti-phospholipase A₂ (anti-PLA₂) de venins de serpents, caractérisés en ce qu'ils sont constituées par des anticorps monoclonaux polyspécifiques, c'est-à-dire capables de reconnaître non seulement la PLA₂ utilisée pour les préparer, mais également au moins une autre PLA₂ toxique de venins de serpents des familles des Elapidés et des Hydrophidés, en ce que lesdits anticorps sont de type IgG₁,K, en ce qu'ils présentent une grande affinité pour la notexine, exprimée par leur constante de dissociation, $K_D$, comprise entre $1,4 \times 10^{-9}M$ et $15,8 \times 10^{-9}M$ et en ce qu'ils sont obtenus par clonage d'hybridomes resultant de la fusion de cellules myélomateuses appropriées et de cellules spléniques immunisées par un antigène constitué par une phospholipase A₂ (PLA₂) de venins de serpents des familles des Elapidés et des Hydrophidés, notamment la notexine et la PLA₂ basique de Naja nigricollis, pour laquelle les Inventeurs ont attribué le nom de nigexine, en mettant en oeuvre une technique de clonage appropriée.

Selon un autre mode de réalisation avantageux desdits anticorps, ceux-ci sont obtenus par clonage d'hybridomes résultant de la fusion de cellules myélomateuses appropriées et de cellules spléniques doublement immunisées, c'est-à-dire immunisées par deux PLA₂ de venins de serpents des familles des Elapidés et des Hydrophidés, l'immunisation étant réalisée soit par injection d'un mélange de ces deux PLA₂, soit par administration séparée de ces deux PLA₂ successivement.

Les anticorps monoclonaux conformes à l'invention sont avantageusement obtenus, d'une manière connue en elle-même, par fusion de cellules spléniques de souris immunisées par un antigène constitué par au moins une phospholipase A₂ (PLA₂) de venin de serpent des familles des Elapidés et des Hydrophidés avec des cellules myélomateuses appropriées, de préférence d'un myélome non sécrétant et de préférence encore en présence de polyéthylèneglycol ; les hybridomes obtenus, qui sécrètent des anticorps anti-PLA₂ de venins de serpents des familles des Elapidés et des Hydrophidés, sont alors clonés de manière à fournir les anticorps monoclonaux anti-PLA₂ poly-spécifiques conformes à la présente invention, qui sont recueillis et purifiés.

L'immunisation est réalisée en deux étapes successives en utilisant notamment des antigènes constitués par la notexine du venin de Notechis scutatus scutatus et la nigexine de Naja nigricollis, encore que l'on puisse envisager de ne réaliser l'immunisation qu'avec un seul de ces antigènes.

Les hybridomes clonés peuvent être injectés à des mammifères appropriés, notamment des souris, chez lesquelles ils développent des ascites de première génération riches en anticorps monoclonaux, qui sont avantageusement réinjectées à d'autres souris chez lesquelles ces ascites de première génération forment des ascites de deuxième génération, encore enrichies en anticorps monoclonaux, lesquels sont séparés de ces liquides d'ascite par tous moyens appropriés, tels que centrifugation, filtration.

La présente invention a également pour objet des fragments antigéniques, caractérisés en ce qu'ils sont reconnus par les anticorps monoclonaux polyspécifiques conformes à l'invention, et en ce que leurs séquences n'appartiennent pas au site toxique ou au site de reconnaissance des surfaces lipidiques des PLA₂ de venins des serpents des familles des Elapidés et des Hydrophidés.

Parmi ces fragments antigéniques, l'on peut citer en particulier :
- Un fragment antigénique, caractérisé en ce qu'il présente la séquence suivante en amino-acides :
G-S-G-T-P-V-D-E-L-D-R-C-C-K-I-H-D-D-C-Y-D-E-A-G-K-K
et en ce qu'il correspond à une fraction du site antigénique d'une pluralité de PLA₂ de venins de serpents des familles des Elapidés et des Hydrophidés.
- Un fragment antigénique, caractérisé en ce qu'il présente la séquence suivante en amino-acides :
W-N-I-D-T-K-K-R-C-Q
et en ce qu'il correspond à une fraction du site antigénique d'une pluralité de PLA₂ de venins des serpents des familles des Elapidés et des Hydrophidés.

Conformément à l'invention, lesdits fragments antigéniques sont notamment des fractions du site antigénique des PLA₂ des venins de Notechis ater kangaroo, Notechis ater ater, Notechis ater serventyi, Notechis occidentalis, Pseudonaja nuchalis, Pseudonaja butleri, Pseudechis porphyriacus, Pseudechis papuanus, Micropechis ikehaka, Hoplocephalus stephensii, Acantophis antarticus, Acontophis barkley, Oxyuranus scutellatus, Laticauda colubrina, Laticauda laticaudata, Aipysurus laevis, Naja melanoleuca, Naja melanoleuca subgulva, Naja haje, Naja oxiana, Naja kaouthia (siamensis), Hemachatus haemachatus, Naja nigricollis nigricollis, Naja nivea, Naja mossambica nigricincta, Naja mossambica katiensis, Naja naja atra.

La présente invention a aussi pour objet un réactif immunologique utilisable pour la détection dans des fluides biologiques d'une ou plusieurs PLA₂ de venins de serpents, caractérisé en ce qu'il est constitué par des anticorps monoclonaux polyspécifiques conformes à l'invention.

Selon un mode de réalisation avantageux dudit réactif immunologique, celui-ci comprend lesdits anticorps monoclonaux seuls, en mélange et/ou combinés à d'autres anticorps notamment antineurotoxine et/ou anticardiotoxine, fixés ou non sur des supports solides.

La présente invention a également pour objet un procédé de détection d'empoisonnement par un venin de serpent des familles des Elapidés et des Hydrophidés, qui consiste à détecter une phospholipase A₂ présente dans un fluide biologique tel que sang, sérum ou venin à contrôler, à l'aide des anticorps monoclonaux polyspécifiques conformes à l'invention, en mettant en présence ledit fluide biologique avec le réactif immunologique conforme à l'invention, auquel se lient les PLA₂, si de tels antigènes sont présents dans l'échantillon de fluide biologique à contrôler, la lecture du résultat étant révélée par un moyen approprié notamment RIA, EIA.

3

La présente invention a en outre pour objet un kit prêt à l'emploi, pour la détection dans un fluide biologique ou dans un venin à contrôler, de PLA$_2$ de venins de serpents de la famille des Elapidés ou des Hydrophidés, caractérisé en ce qu'il comprend :

- des doses appropriées d'anticorps monoclonaux polyspécifiques dirigés contre les PLA$_2$ de venins de serpents de la famille des Elapidés ou des Hydrophidés, fixés sur un support solide approprié.

- des doses appropriées de notexine de Notechis scutatus scutatus et/ou de nigexine de Naja nigricollis couplées à un système enzymatique approprié tel que avidine - biotine

- acétylcholine estérase ou phosphatase alcaline -paranitrophénylphosphate, de révélation de la réaction anticorps-PLA$_2$.

- des quantités utiles de tampons appropriés pour la mise en oeuvre de ladite détection.

Outre les dispositions qui précèdent, l'invention comprend encore d'autres dispositions, qui ressortiront de la description qui va suivre, qui se réfère à un exemple de préparation d'anticorps monoclonaux antiphospholipase A$_2$ de venin de serpent de la famille des Elapidés ou des Hydrophidés, à des essais mettant en évidence l'action polyspécifique des anticorps monoclonaux selon l'invention, ainsi qu'à leurs effets sur l'activité enzymatique de la PLA$_2$ in vitro ainsi que sur la toxicité de celle-ci in vivo.

Il doit être bien entendu, toutefois, que ces exemples et les parties descriptives correspondantes, sont donnés uniquement à titre d'illustration de l'objet de l'invention, dont ils ne constituent en aucune manière une limitation.

**Exemple 1 : Préparation des anticorps monoclonaux polyspécifiques anti-PLA$_2$ de venins de serpents des familles des Elapidés et des Hydrophidés.**

1°) Immunisation :

- Immunisation simple contre la notexine

Les souris sont de souche BALB/C. Les animaux sont immunisés selon le protocole résumé dans le tableau I ci-dessous.

TABLEAU I

| JOURS | 0 | + 7 | + 14 | + 21 | + 25 | + 40 | + 55 | + 70 | + 85 |
|---|---|---|---|---|---|---|---|---|---|
| Injection Intravei-neuse | 0.25 μg Ser Ø | | | | | | | | |
| Injection souscu-tanée | | 0.3 μg Ser Ø | 0.5 μg + Ser Ø | 0.5 μg + Ser Ø | 10 μg + Freund | 30 μg + Freund | 50 μg + Freund | 75 μg + Freund | |
| Injection Intrapéri-tonéale : BOOST | | | | | | | | | 10 μg + Ser Ø |

Au 88ème jour, on effectue la fusion cellulaire ou un injection de cellules d'Ehrlich.

Lors des quatre premières injections, la notexine est dissoute dans du sérum physiologique. Lors des injections suivantes, la notexine dissoute dans le sérum physiologique, est émulsionnée avec de l'adjuvant de Freund incomplet (DIFCO) en proportions 5/3 : sérum physiolo gique/adjuvant. Les volumes d'injection varient entre 50 et 200 μl.

Pour tester la quantité d'anticorps apparue, le sang est prélevé dans la veine de la queue des souris.

- Immunisation double : contre la PLA$_2$ basique (nigexine) de Naja nigricollis et la notexine.

Le protocole est résumé dans le tableau II ci-après.

| JOURS | 0 | + 14 | + 28 | + 43 | + 57 | + 70 | + 76 | + 79 | + 86 | + 93 |
|---|---|---|---|---|---|---|---|---|---|---|
| Injection IV Notexine | | | | | | 0.3 µg Ser ø | | | | |
| Notexine injection s/cutanée | | | | | | | | 0.5 µg Ser ø | 0.5 µg Ser ø | 0.5 µg Ser ø |
| PLA$_2$ Naja nigricollis (injection s/cutanée) | 200 µg ( + Freund complet) | 200 µg ( + Fr.ic) 50 % | 400 µg Fr.ic 50 % | 100 µg (Ser ø) | 200 µg (Ser ø) | | 200 µg Ser ø | | | |

| SUITE | + 105 | + 115 | + 125 | + 140 | 147 | 150 | 153 |
|---|---|---|---|---|---|---|---|
| Notex IV | | | | | | | |
| Notex SC. | 10 µg (37%) + Fr. ic | 20 µg + Fr.ic 37% | 30 µg Fr.ic 37% | 50 µg Fr.ic 37% | 70 µg Fr.ic 37% | | |
| PLA$_2$ SC. | | | | | | | |
| Notex IP (Rappel) | | | | | | 5 µg Ser ø | FUSION |

L'immunisation débute avec la PLA$_2$ de Naja nigricollis, et s'achève avec la notexine. L'adjuvant de Freund, ajouté à la PLA$_2$ de Naja nigricollis, l'est, dans une proportion de 50 %. L'adjuvant de Freund, incomplet, ajouté à la notexine, l'est dans une proportion de 37 %.

2°) Obtention d'anticorps monoclonaux

Les souris sont immunisées selon le protocole exposé ci-dessus. La quantité d'anticorps présente dans le sérum est titrée en radio-immuno essai (RIA). Trois à quatre jours après la dernière injection (10 µg), deux animaux sont sacrifiés pour la fusion cellulaire.

- Fusion :

Les rates, prélevées stérilement dans un milieu DMEM, ou RPMI, sont dilacérées. Les cellules spléniques sont fusionnées avec les cellules myélomateuses, au moyen de PEG 4000 à 50 %, selon le protocole décrit par Kohler et Milstein (1975). Les proportions cellules spléniques/cellules myélomateuses sont de 10 pour une souris, de 2 pour l'autre souris. Après élimination du PEG, la totalité des cellules est reprise dans du milieu sélectif HAT, et répartie dans des boîtes de culture de 24 puits, à raison de 250 µl par puits (environ 5 x 10$^5$ cellules par puits).

Les cellules sont cultivées en présence de milieu sélectif HAT jusqu'à disparition des cellules myélomateuses non hybridées. Les puits sont testés tous les deux jours en RIA, afin de localiser ceux qui contiennent des hybrides sécréteurs d'anticorps anti-notexine.

- Clonage :

Les puits positifs sont clonés par dilution limite. La quantité totale de cellules est déterminée par comptage sur une cellule de Malassez d'une dilution, après coloration au bleu trypan. Les cellules sont réparties dans des boîtes de culture de 96 puits, à raison de 50 µl par puits, contenant de 1 à 0,3 cellule par puits.

Les clones sont cultivés avec du milieu HAT à 15 % de SVF, et testés régulièrement en RIA.

Les hybrides sont repiqués de puits en puits sur un lit de macrophages intrapéritonéaux préparés à partir de souris congéniques Balb/C.

Congélation :

Afin de conserver des cellules hybrides de chaque clone, 10$^7$ cellules sont congelées dans 1 ml de SVF à 7 % de DMSO, dans des cryotubes. La congélation se fait de manière progressive : quelques heures à - 20°C, une nuit à -80°C, puis dans l'azote liquide.

- Ascites :

Les cellules hybrides issues d'un clone sécréteur sont injectées intrapéritonéalement, dans des souris histocompatibles Balb/C. Environ 5 x 10$^6$ cellules hybrides sont injectées par souris. Les souris ont été auparavant déprimées du point de vue immunitaire par injection de pristane, afin d'éliminer le risque de rejet. On peut également utiliser des souris de race nude, dont le système immunitaire est naturellement déprimé.

Les hybrides de cellules spléniques et de cellules myélomateuses développent une ascite chez les souris. Ce liquide d'ascite, riche en anticorps monoclonaux, est dit de "première génération". 200 µl de ce liquide, injectés à d'autres souris, donne un ascite dit de "deuxième génération.

- Conservation des ascites :

Les ascites sont conservées à - 20°C avec de l'azide de sodium 0,1 %. Après décongélation, ils sont laissés 24 h à 4°C pour permettre la coagulation de la fibrine, puis centrifugés ou filtrés.

3°) Purification des anticorps à partir des liquides d'ascite.

- Précipitation au sulfate d'ammonium.

Toutes les étapes de la purification sont effectuées à 4°C ou dans la glace. Après centrifugation des ascites à 10.000 rpm pendant 30 mn, et filtration sur fritté n° 2, les ascites sont additionnés d'une solution saturée de sulfate d'ammonium dans un rapport de volume tel que la concentration finale en sulfate d'ammonium soit de 40 %. La précipitation se poursuit pendant 12 heures, puis le mélange est centrifugé à 8.000 rpm pendant 25 minutes.

Le culot de précipitation est repris dans une solution de sulfate d'ammonium à 40 %, et centrifugé une seconde fois à 8.000 rpm pendant 15 minutes. Le culot est repris dans un volume minimum de tampon phosphate de Na 50 mM pH = 7 contenant 0,9 % de NaCl. Il est dialysé au moins deux fois 24 heures contre le même tampon.

- Purification sur colonne d'affinité.

La colonne d'affinité est formée par un gel de sépharose 4B, sur lequel est greffée de la notexine. 5mg de notexine, dissous dans 5 ml de tampon NaHCO$_3$ 0,1 M, 0,5 M NaCl pH = 8, sont ajoutés à 1 g de gel sépharose 4B activé au bromure de cyanogène, préalablement hydraté dans HCl 10$^{-3}$ M, et lavé par le tampon NaHCO$_3$.

Le mélange est laissé 2 heures à température ambinate, et 24 heures à 4°C, sous agitation. Puis l'excès de groupements réactifs du gel est saturé par une solution 1 M de glycine pendant 2 heures à température ambiante.

Le gel est déposé sur une colonne, et trois cycles de lavage sont effectués avec les tampons qui seront utilisés pour la purification des anticorps :
. Tampon : Tris HCl 0,1 M NaCl 0,5 M pH = 8
. Tampon : HCl-glycine 0,1 M NaCl 0,5 M pH = 2,5.

La colonne est disposée dans une chambre froide à 4°C. Le liquide d'ascite, après filtration sur fritté taille 3 est déposé sur la colonne équilibrée à pH = 8. Il est élué lentement, et un deuxième passage est effectué à pH = 8. Ainsi l'association des anticorps avec la toxine greffée sur le gel a le temps de se faire. Puis la colonne est lavée à pH = 8 jusqu'à l'obtention d'une absorbance nulle de l'éluat à 280 nm. Seuls les anticorps spécifiques associés à la toxine, demeurent sur le gel, et sont élués par choc de pH avec le tampon glycine à pH = 2,5. Dès la sortie de la colonne, ils sont recueillis dans un tampon Tris 1 M pH = 8, puis dialysés contre un tampon Tris 0,1 M pH = 8, ou concentrés par filtration sous vide sur membrane, au moyen d'une cartouche CX 30 millipore.

- Chromatographie haute pression en échange d'ions.

Afin d'apprécier la pureté des anticorps monoclonaux obtenus après précipitation au sulfate d'ammonium, un aliquot est chromatographié sur une colonne analytique échangeuse d'anions faible, de type PVDI (Société française de chromatographie), en HPLC. Le système de solvants est :
. Solvants A : tampon tris 20 mM - acétate, pH = 7,9
. Solvants B : tampon tris 20 mM $Na_2SO_4$ 0,15 M - acétate pH = 7,9.

Le débit est de 0.5 ml/min.

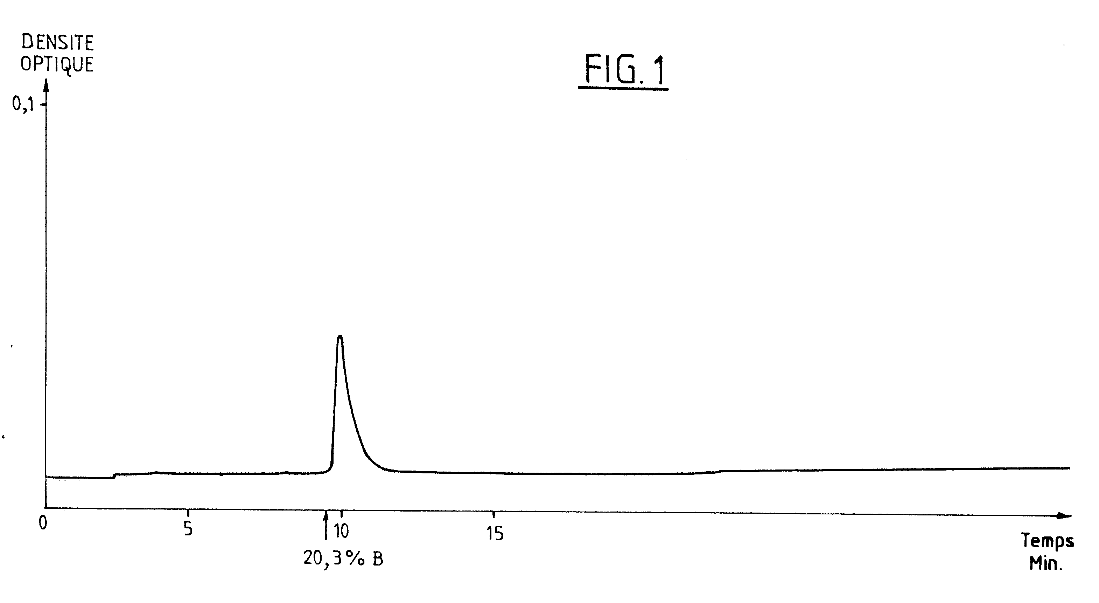

La figure 1 représente le chromatogramme des anticorps monoclonaux polyspécifiques $MN_1$ purifiés ; sur cette figure, on a en abscisse le temps en minutes et en ordonnée la densité optique.

4°) Titrage des anticorps.

100 µl de solutions d'anticorps de dilutions croissantes sont mis en présence de 100 µl d'une concentration constante de notexine iodée. 100 µl de tampon PBS sont ajoutés dans chaque tube. La lecture est réalisée au moyen d'un radioimmunoessai (RIA) effectué soit par la méthode au polyéthylèneglycol, soit par la méthode par double anticorps.

Les titres des solutions d'anticorps, obtenues après purification sont :
$1/5 \times 10^4$, $1/10^3$ pour $MN_1$ et $MN_2$ respectivement (RAS notexine : $5,5 \times 10^{15}$ Bq/mole).

5°) Détermination de la classe des anticorps monoclonaux.

Elle est réalisée par une technique de double diffusion en gel dite Ouchterlony.

Six puits de diamètre 1 mm sont disposés sur un cercle autour d'un puits central de même diamètre. Le puits central reçoit un antisérum : sérum anti Ig G, anti Ig A, anti Ig M, anti Ig $G_1$, anti IgG $_{2a}$, anti IgG $_{2b}$. Les puits périphériques reçoivent une ou plusieurs dilutions du liquide d'ascite à analyser. La migration se poursuit une nuit en atmosphère humide. La révélation se fait par coloration des arcs de précipitation, selon une technique analogue à celle de l'isoélectrophorèse. Les anticorps $MN_1$ et $MN_2$ sont de type Ig $G_1$, K.

## Exemple 2: Effet des anticorps monoclonaux conformes à l'invention sur l'activité enzymatique de la notexine.

La mesure de l'activité enzymatique est réalisée à l'aide d'un appareil pH stat (Methrom). Les acides gras libérés sont titrés à pH constant (pH = 8), par une solution d'hydroxyde de Na 0,01 N à 40°C.

Le substrat est une émulsion de phosphatidylcholine (Halpert - Eaker, 1975), dans une solution de $CaCl_2$ 20 mM, NaCl 0,12 M, en présence de désoxycholate de sodium.

Le rapport concentration finale de phosphatidylcholine/concentration finale de désoxycholate de sodium est maintenu constant, égal à 3,7.

Les solutions initiales sont : phosphatidylcholine : 600 mM dans l'éthanol, désoxycholate de sodium : 270 mM dans $H_2O$. Le volume réactionnel final est de 4 ml.

Les volumes de soude ajoutés pour maintenir le pH constant sont enregistrés automatiquement en fonction du temps.

Les anticorps monoclonaux sont dialysés pendant 24 heures dans la solution de $CaCl_2$ utilisée pour effectuer les mesures, afin d'éviter une modification du pH du milieu réactionnel, lors de l'addition des anticorps. Ils sont incubés avec la notexine pendant une heure à température ambinate.

L'activité enzymatique de la notexine ainsi traitée, est alors mesurée.

L'analyse cinétique de la réaction enzymatique a été faite par deux méthodes : mesure de $V_i$ et mesure de $V_{max}$.

Pour l'étude de $V_i = f([S])$ et $1/V_i = f(1/[S])$, à concentration de notexine constante ($[E_t] = 0,5$ µg/4 ml), la concentration de substrat varie de 7,5 mM à 82,5 mM. La mesure est réalisée en absence et en présence d'anticorps monoclonaux polyspécifiques $MN_1$, en excès molaire de 10 par rapport à la notexine.

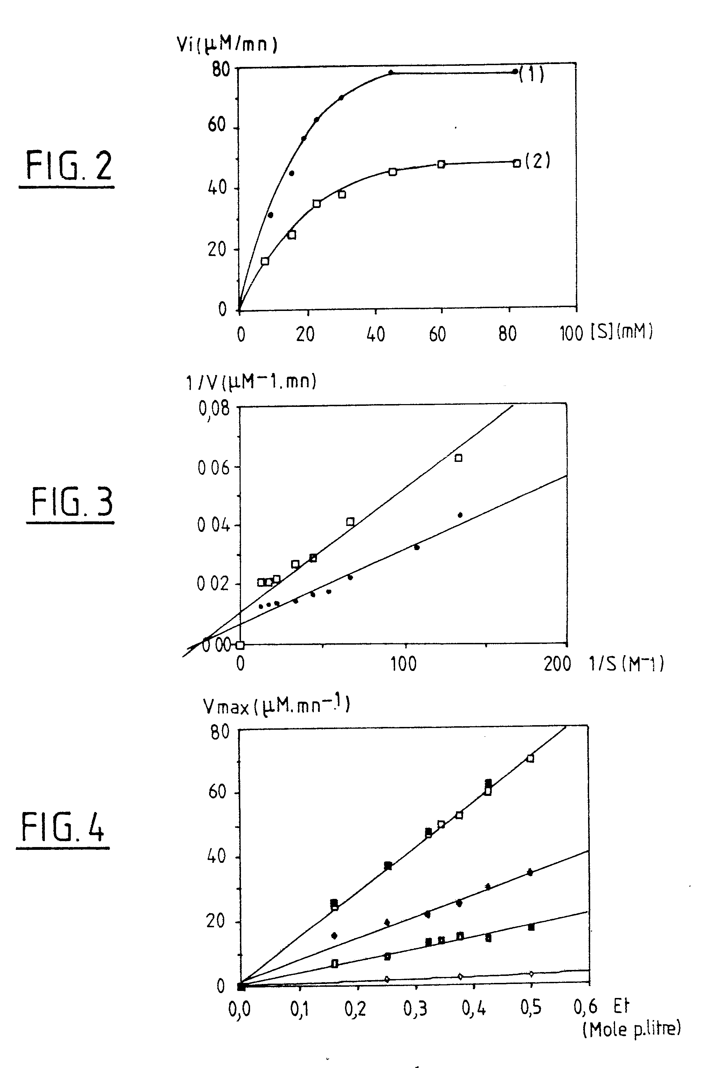

Les courbes obtenues en présence et en absence d'anticorps sont représentées sur la figure 2, qui comporte en abscisse la concentration en substrat en mM et en ordonnées la vitesse initiale ($V_i$) en µM/mn, la courbe 1 (♦) correspond à la notexine seule ; la courbe 2 (□) correspond à la notexine en présence

d'anticorps $MN_1$.

L'allure hyperbolique de ces courbes indique que la notexine suit une cinétique de type michaélien.

Si l'on trace $1/V_i$ en fonction de $1/[S]$, dans le cas de la notexine, on obtient bien une droite, en présence comme en absence d'anticorps ; la figure 3 est une représentation de Linweaver-Burk et comporte en abscisse $1/[S]$, en $M^{-1}$ et en ordonnée $1/V_i$ en $\mu M^{-1}$.mn. Il est aisé de déterminer graphiquement $K_M$ et $V_{max}$ à partir de ces droites ($-1/K_M$ et $1/v_{max}$ sont donnés respectivement par l'abscisse et l'ordonnée à l'origine).

Pour la notexine seule (♦) :

$K_m = 0,034\ M = 34\ mM$

$V_{max} = 128\ \mu M \times mn^{-1}$

Pour la notexine en présence d'anticorps $MN_1$ (□) :

$K_M = 0,034\ M = 34\ mM$

$V_{max} = 88\ \mu M \times mn^{-1}$

Il en résulte qu'en présence d'anticorps, la vitesse maximale est diminuée, sans modification du $K_M$.

Les anticorps ne modifiant pas le $K_M$, ils ne modifient donc pas l'affinité de l'enzyme pour le substrat ; ils répondent au modèle des inhibiteurs non compétitifs : ils se lient à l'enzyme indépendamment du substrat pour former un complexe ternaire enzyme-substrat-anticorps (ou ESI).

Les résultats obtenus permettent donc de proposer un modèle cinétique pour illustrer l'effet provoqué par les anticorps monoclonaux sur l'activité enzymatique de la notexine : modèle d'inhibition non compétitive partielle.

Cette méthode permet également de déterminer la concentration de substrat pour laquelle $V = V_{max}$.

On a étudié les variations de la vitesse de réaction, en fonction de la quantité d'enzyme : $0 < E_t < 0,5\ \mu g$ pour $[S] = 45\ mM$, en absence et en présence d'anticorps monoclonaux $MN_1$, $MN_2$, des anticorps polyclonaux, et d'anticorps monoclonaux anti-cardiotoxine. Les résultats sont présentés figure 4, qui montre l'influence des différents anticorps sur l'activité enzymatique de la notexine ((□) notexine seule, (♦) notexine + $MN_1$, (▣) notexine + $MN_2$, (◊) notexine + anticorps polyclonaux, (■) notexine + anticorps anti-cardiotoxine). Cette figure 4 comporte en abscisse la quantité d'enzyme $E_t$ en mole par litre et en ordonnée la vitesse maximale ($V_{max}$), en $\mu M.mn^{-1}/0,5$.

On peut voir que, bien que la vitesse représente 60 % de la vitesse maximale, la relation vitesse : $f([E_t])$ est linéaire dans la gamme de concentrations d'enzyme utilisée.

On peut donc calculer les activités enzymatiques en absence et en présence des différents types d'anticorps. Le tableau III ci-après précise ces activités enzymatiques.

TABLEAU III

| | Activité enzymati-que ($\mu M \times$ mn-1/0,5 $\mu g$ enzyme) | % Activité résiduelle | % inhibition de l'activité |
|---|---|---|---|
| Notexine | 70 | 100 | 0 |
| Notexine + $MN_1$ | 34,5 | 49,3 | 50,7 |
| Notexine + $MN_2$ | 17,5 | 25 | 75 |
| Notexine + POLYCLONAL | 2,85 | 4 | 96 |
| Notexine + AC à cardiotoxine | 70 | 100 | 0 |

L'anticorps monoclonal non spécifique de la notexine (anticardiotoxine), n'a aucun effet sur l'activité enzymatique de la notexine.

Les anticorps polyclonaux inhibent 96 % de l'activité enzymatique, tandis que les anticorps monoclonaux $MN_1$ et $MN_2$ inhibent partiellement cette activité : de 50,7 et 75 % respectivement.

**Exemple 3 : Détermination de l'affinité des anticorps monoclonaux pour la notexine.**

L'affinité des anticorps monoclonaux $MN_1$ et $MN_2$, pour la notexine, a été calculée selon la méthode décrite par Scatchard (1949).

Des quantités croissantes de traceur radioactif sont mises en présence d'une quantité fixe d'anticorps monoclonaux correspondant au titre, dans un volume total de 300 µl :

traceur radioactif : 100 µl

solution d'anticorps diluée au titre : 100 µl tampons PBS : 100 µl.

On obtient ainsi la quantité de radioactivité totale liée.

Parallèlement, une série de tubes contient :

traceur radioactif : 100 µl

solution d'anticorps au titre : 100 µl

solution de notexine froide en excès molaire de 800 par rapport au traceur : 100 µl.

On obtient ainsi la quantité de radioactivité liée de façon non spécifique.

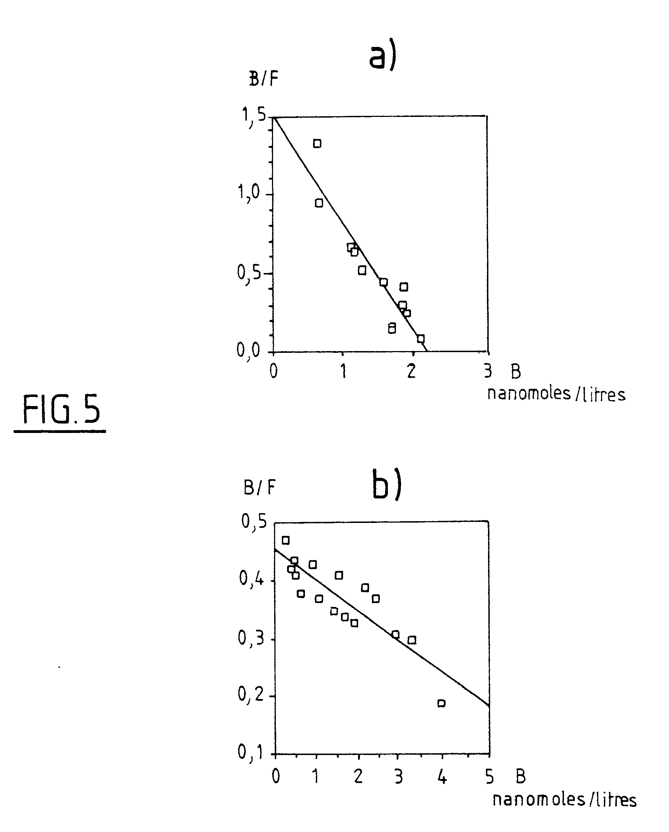

La figure 5 montre l'affinité des anticorps monoclonaux $MN_1$ (a) et $MN_2$ (b) pour la notexine. Cette figure comporte en abscisse la fraction de radioactivité liée aux anticorps de façon spécifique B, en nanomoles/litre et en ordonnée le rapport B/F, dans lequel B est tel que défini ci-dessus et F correspond à la fraction de radio-activité libre.

La constante de dissociation $K_D$ est inversement proportionnelle à l'affinité de l'anticorps.

Les valeurs des $K_D$, déterminées graphiquement, sont : $1,4 \times 10^{-9}$ M et $15,8 \times 10^{-9}$ M, respectivement/pour $MN_1$ et $MN_2$.

Une méthode de calcul indirecte décrite par Müller (1980) donne la constante d'équilibre en fonction de la concentration inhibitrice 50 : $K_D = 3/8$ (I - T)

$I = IC_{50}$

T = concentration en traceur radioactif.

Les valeurs des $IC_{50}$ trouvées pour $MN_1$ et $MN_2$ donnent un $K_D$ de $5,3 \times 10^{-9}$ M et de $8 \times 10^{-9}$ M, respectivement pour $MN_1$ et $MN_2$. Ces valeurs des $K_D$ sont compatibles avec celles obtenues précédemment selon Scatchard.

## Exemple 4 : Toxicité de la notexine in vivo, en présence d'anticorps.

Les préparations d'anticorps monoclonaux ($MN_1$ et $MN_2$) utilisées pour cette étude proviennent d'une précipitation au sulfate d'ammonium de liquides d'ascites de souris, suivie de trois dialyses successives de 24 heures, contre du tampon tris 20 mM pH = 7,7.

La concentration des solutions d'anticorps monoclonaux a été évaluée d'après leur titre, par la formule :

$A_0 = D_D + TO/2$ où $A_0$ = concentration d'anticorps présente dans le RIA à la dilution correspondant au titre.

$K_D$ = constante de dissociation de l'anticorps.

T0/2 = concentration de traceur fixé par les anticorps au titre. C'est-à-dire la moitié de la concentration totale de traceur fixable par les anticorps.

Les concentrations en anticorps obtenues sont :

$5,4 \times 10^{-4}$ M pour $MN_1$

$4,2 \times 10^{-5}$ M pour $MN_2$

- Les injections sont faites par voie intraveineuse ans la queue de souris de 20 ± 2 g. Les souris sont examinées 24 heures après injection.

- Pour chaque anticorps, les souris (au moins trois), reçoivent un mélange de notexine en quantité légèrement supérieure à la $DL_{100}$ (soit 0,6 µg par souris de 20 g), et d'anticorps en excès d'au moins 100 en mole, préincubé une nuit à 4°C. La solution de notexine utilisée est dans du sérum physiologique. (L'excès de 100, en mole, correspond à un excès de 200 en sites, si l'on considère qu'une molécule d'anticorps comporte deux sites anti-notexine).

- Parallèlement, des souris témoins reçoivent la même quantité de notexine seule, dans le même volume que précédemment (qsp tampon tris 20 mM pH = 7,4).

- Un troisième lot de souris, reçoit, toujours dans le même volume, la même quantité d'anticorps seuls (qsp sérum physiologique). On vérifie ainsi l'innocuité des solutions d'anticorps, pour les animaux.

- L'étude d'un effet synergique éventuel entre les anticorps monoclonaux pris à deux, suit un protocole analogue, la notexine est cette fois incubée avec les deux anticorps simultanément, chacun en excès de 100 en mole. A titre de comparaison, d'autres souris reçoivent la même quantité de notexine avec l'un, ou l'autre anticorps séparément (toujours en excès de 100).

Les résultats sont présentés dans le tableau IV ci-après, où les chiffres indiqués correspondent au pourcentage de survie des souris, 24 heures après injection.

TABLEAU IV

| | AC | AC Polyclonaux | MN$_1$ | MN$_2$ | | MN$_1$ + MN$_2$ | |
| Type d'injection | | | | | | | |
| Notexine + AC | | 100 | 0 | 0 | | 0 | |
| Témoin Notexine + Tris | | 0 | 0 | 0 | | 0 | |
| Témoin AC + sérum Ø | | 100 | 100 | 100 | | 100 | |

Les résultats indiquent clairement que les anticorps polyclonaux ont un effet neutralisant in vivo, alors que tous les anticorps monoclonaux étudiés ici en sont dépourvus. De plus, on n'observe aucun synergie entre ces anticorps monoclonaux pris deux à deux.

L'effet neutralisant des anticorps polyclonaux, qui semble général, est confirmé dans le cas de la notexine. Quant aux anticorps monoclonaux, il n'a pas été dé crit, jusqu'à présent, dans la littérature, d'anticorps monoclonaux neutralisants contre une phospholipase toxique. Des trois anticorps monoclonaux obtenus contre la bungarotoxine détoxifiée (STRONG P.N. et al., Eur. J. Biochem., 1984, 142, 145 - 151), aucun n'est neutralisant in vivo.

**Exemple 5 : Etude des réactions antigéniques croisées et identification d'une zone antigénique reconnue par l'anticorps monoclonal.**

Les résultats sont présentés dans le tableau V ci-après. Contrairement aux anticorps polyclonaux, très spécifiques de la notexine, les anticorps monoclonaux polyspécifiques conformes à l'invention, MN$_1$ et MN$_2$, ont la particularité d'avoir un très large spectre de reconnaissance. On remarque que trois Elapidés (Dendroaspis angusticeps, Dendroaspis polylepis, Dendroaspis viridis) ne présentent pas de réaction croisée ; ceci est dû au fait qu'ils ne possèdent pas de PLA$_2$ dans leurs venins. On note également que les Vipéridés et les Crotalidés (Vipera aspis, Vipera russelli, Vipera russelli pulchella, Agkistrodon rhodostoma, Bitis arietans, Crotalus adamenteus) ne présentent pas de réaction croisée.

TABLEAU V

Résultats des réactions de compétition pour

les anticorps monoclonaux MN1, MN2.

| TOXINES NON PHOSPHOLIPASIQUES PURIFIEES | $IC_{50}$ $(10^{-8}M)$ | |
| --- | --- | --- |
| | MN1 | MN2 |
| - neurotoxine Naja nigricollis | > 1000 | > 1000 |
| - cobratoxine (ou toxine III) de Naja siamensis | > 1000 | > 1000 |

| PLA2 PURIFIEES | $IC_{50}$ $(10^{-8}M)$ | |
| --- | --- | --- |
| | MN1 | MN2 |
| Notexine | 1,8 | 2,3 |
| Notechis 2-5 | 8,0 | 8,0 |
| Notechis 2-1 | 50,0 | > 200 |
| PLA2 basique Naja nigricollis | 0,65 | 2 |
| Fragment I9 - Q118 de PLA2 Naja nigricollis | 0,86 | 2,2 |
| PLA2 acide Naja nigricollis | 12 | 75 |
| PLA2 cmIII de Naja mossambica mossambica | 0,65 | 1 |
| PLA2 cmII de Naja mossambica mossambica | 2,5 | 15 |
| bungarotoxine | > 1000 | |
| Crotoxine, sous unité CA | > 1000 | > 1000 |
| Crotoxine, sous unité CB | > 1000 | > 1000 |
| PLA2 de Crotalus durissus durissus | > 1000 | > 1000 |
| PLA2 de Crotalus adamenteus | > 1000 | > 1000 |
| PLA2 venin d'abeille | > 1000 | > 1000 |
| PLA2 pancréas porc | > 1000 | > 1000 |
| PLA2 intracellulaire de macrophage | > 1000 | > 1000 |
| PLA2 intracellulaire de lymphocyte | > 1000 | > 1000 |

## TABLEAU V (suite I)

| VENINS COMPLETS | Présence de réaction croisée | |
|---|---|---|
| | MN$_1$ | MN$_2$ |
| Notechis ater kangaroo | + | + |
| Notechis ater ater | + | + |
| Notechis ater serventyi | + | + |
| Notechis occidentalis | + | + |
| Pseudonaja nuchalis | + | + |
| Pseudonaja butleri | + | + |
| Pseudechis porphyriacus | + | + |
| Pseudechis papuanus | + | + |
| Micropechis ikehaka | + | + |
| Hoplocephalus stephensii | + | + |
| Acantophis antarticus | + | + |
| Acantophis barkley | + | + |
| Oxyuranus scutellatus | + | + |
| Laticauda colubrina | + | + |
| Laticauda laticauda | + | + |
| Aipysurus laevis | + | + |
| Naja melanoleuca | + | + |
| Naja melanoleuca subgulva | + | + |
| Naja haje | + | + |
| Naja oxiana | + | + |
| Naja kaouthia (siamensis) | + | + |
| Hemachatus haemachatus | + | + |
| Naja nigricollis nigricollis | + | + |
| Naja nivea | + | + |
| Naja mossambica nigricincta | + | + |

TABLEAU V (suite II)

| VENINS COMPLETS | Présence de réaction croisée | |
|---|---|---|
| | MN₁ | MN₂ |
| Naja mossambica katiensis | + | + |
| Naja naja atra | + | + |
| Dendroaspis angusticeps | − | |
| Dendroaspis polylepis | − | |
| Dendroaspis viridis | − | |
| Vipera aspis | − | |
| Vipera russelli | − | |
| Vipera russelli pulchella | − | |
| Agkistrodon rhodostoma | − | |
| Bitis arietans | − | |
| Crotalus adamenteus | − | |

Les anticorps MN₁ et MN₂ préparés à partir d'animaux doublement immunisés, reconnaissent non seulement les deux PLA₂ utilisées pour l'immunisation, mais aussi une large famille d'autres PLA₂ toxiques.

Ainsi que cela ressort de ce qui précède, l'invention ne se limite nullement à ceux de ses modes de réalisation et d'application qui viennent d'être décrit de façon explicite ; elle en embrasse au contraire toutes les variantes qui peuvent venir à l'esprit en la matière sans s'écarter du cadre, ni de la portée de la présente invention.

## Revendications

1°) Anticorps monoclonaux anti-phospholipase A₂ (anti-PLA₂) de venins de serpents, caractérisés en ce qu'ils sont constitués par des anticorps monoclonaux polyspécifiques, c'est-à-dire capables de reconnaître non seulement la PLA₂ utilisée pour les préparer, mais également au moins une autre PLA₂ toxique de venins de serpents des familles des Elapidés et des Hydrophidés, en ce que lesdits anticorps sont de type IgG₁,K, en ce qu'ils présentent une grande affinité pour la notexine, exprimée par leur constante de dissociation, $K_D$, comprise entre $1,4 \times 10^{-9}$M et $15,8 \times 10^{-9}$M et en ce qu'ils sont obtenus par clonage d'hybridomes résultant de la fusion de cellules myélomateuses appropriées et de cellules spléniques immunisées par un antigène constitué par une phospholipase A₂ (PLA₂) de venins de serpents des familles des Elapidés et des Hydrophidés, notamment la notexine de Notechis scutatus scutatus et la PLA₂ basique de Naja nigricollis, dénommée nigexine, en mettant en oeuvre une technique de clonage appropriée.

2°) Anticorps selon la revendication 1, caractérisés en ce qu'ils sont obtenus par clonage

13

EP 0 320 366 A1

d'hybridomes résultant de la fusion de cellules myélomateuses appropriées et de cellules spléniques doublement immunisées, c'est-à-dire immunisées par deux $PLA_2$ de venins de serpents des familles des Elapidés et des Hydrophidés.

3°) Fragments antigéniques, caractérisés en ce qu'ils sont reconnus par les anticorps monoclonaux polyspécifiques selon la revendication 1 ou la revendication 2 et en ce qu'ils n'appartiennent pas au site toxique ou au site de reconnaissance des surfaces lipidiques des $PLA_2$ de venins de serpents des familles des Elapidés et des Hydrophidés.

4°) Fragment antigénique selon la revendication 3, caractérisé en ce qu'il présente la séquence suivante en amino-acides :

G-S-G-T-P-V-D-E-L-D-R-C-C-K-I-H-D-D-C-Y-D-E-A-G-K-K

et en ce qu'il correspond à une fraction du site antigénique d'une pluralité de $PLA_2$ de venins de serpents des familles des Elapidés et des Hydrophidés.

5°) Fragment antigénique selon la revendication 3, caractérisé en ce qu'il présente la séquence suivante en amino-acides :

W-N-I-D-T-K-K-R-C-Q

et en ce qu'il correspond à une fraction du site antigénique d'une pluralité de $PLA_2$ de venins des serpents des familles des Elapidés et des Hydrophidés.

6°) Fragments antigéniques selon l'une quelconque des revendications 3 à 5, caractérisés en ce qu'ils sont notamment des fractions antigéniques de $PLA_2$ des venins de Notechis ater kangaroo, Notechis ater ater, Notechis ater serventyi, Notechis occidentalis, Pseudonaja nuchalis, Pseudonaja butleri, Pseudechis porphyriacus, Pseudechis papuanus, Micropechis ikehaka, Hoplocephalus stephensii, Acantophis antarticus, Acantophis barkley, Oxyuranus scutellatus, Laticauda colubrina, Laticauda laticaudata, Aipysurus laevis, Naja melanoleuca, Naja melanoleuca subgulva, Naja haje, Naja oxiana, Naja kaouthia (siamensis), Hemachatus haemachatus, Naja nigricollis nigricollis, Naja nivea, Naja mossambica nigricincta, Naja mossambica katiensis, Naja naja atra.

7°) Réactif immunologique utilisable pour la détection dans des fluides biologiques d'une ou plusieurs $PLA_2$ de venins de serpents, caractérisé en ce qu'il est constitué par des anticorps monoclonaux polyspécifiques selon l'une quelconque des revendications 1 et 2.

8°) Réactif immunologique selon la revendication 7, caractérisé en ce qu'il comprend lesdits anticorps monoclonaux seuls, en mélange, et/ou combinés à d'autres anticorps notamment antineurotoxine et/ou anticardiotoxine, fixés ou non sur des supports solides.

9°) Procédé de détection d'empoisonnement par un venin de serpent des familles des Elapidés et des Hydrophidés, caractérisé en ce qu'il consiste à détecter la phospholipase $A_2$ présente dans un fluide biologique tel que sang, sérum ou venin à contrôler, à l'aide des anticorps monoclonaux polyspécifiques selon l'une quelconque des revendications 1 et 2, en mettant en présence ledit fluide biologique avec le réactif immunologique selon l'une quelconque des revendications 7 et 8, auquel se lient les $PLA_2$, si de tels antigènes sont présents dans l'échantillon de fluide biologique à contrôler, la lecture du résultat étant révélée par un moyen approprié notamment RIA, EIA.

10°) Kit prêt à l'emploi, pour la détection dans un fluide biologique ou dans un venin à contrôler, de $PLA_2$ de venins de serpents de la famille des Elapidés ou des Hydrophidés, caractérisé en ce qu'il comprend :
- des doses appropriées d'anticorps monoclonaux polyspécifiques dirigés contre les $PLA_2$ de venins de serpents de la famille des Elapidés ou des Hydrophidés selon la revendication 1 ou la revendication 2, fixés sur un support solide approprié.
- des doses appropriées de notexine et/ou de nigexine, couplées à un système enzymatique approprié tel que avidine
- biotine - acétylcholine estérase, ou phosphatase alcaline
- paranitrophénylphosphate, de révélation de la réaction anticorps $PLA_2$.
- des quantités utiles de tampons appropriés pour la mise en oeuvre de ladite détection.

14

FIG. 1

DENSITE OPTIQUE

0,1

20,3 % B

0          5          10          15

Temps Min.

EP 0 320 366 A1

FIG. 2

FIG. 3

FIG. 4

a)

B/F

FIG.5

b)

B/F

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 105, no. 19, 10 novembre 1986, page 355, résumé no. 167934f, Columbus, Ohio; A. KHALIL et al.: "Antigenic relatedness between human lecithin-cholesterol acylstransferase and phospholipases of the A2 family", & BIOCHIM. BIOPHYS. ACTA 1986, 878(1), 127-30 * Résumé * | 1 | C 12 P 21/00<br>C 07 K 7/10<br>C 07 K 7/06<br>G 01 N 33/573<br>G 01 N 33/577//<br>(C 12 P 21/00<br>C 12 R 1:91 ) |
| Y | IDEM --- | 1-10 | |
| Y | CHEMICAL ABSTRACTS, vol. 106, no. 21, 25 mai 1987, page 306, résumé no. 171628x, Columbus, Ohio, US; J.G.N. DE JONG et al.: "Monoclonal antibodies against an intracellular phospholipase A2 from rat liver and their cross-reactivity with other phospholipases A2", & EUR. J. BIOCHEM. 1987, 164(1), 129-35 * Résumé * --- | 1,2,7-10 | |
| Y | CHEMICAL ABSTRACTS, vol. 83, no. 21, 24 novembre 1975, page 186, résumé no. 174219g, Columbus, Ohio, US; J. HALPERT et al.: "Amino acid sequence of presynaptic neurotoxin from the venom of Notechis scutatus scutatus (Australian tiger snake)", & J. BIOL. CHEM. 1975, 250(17), 6990-7 * Résumé * --- -/- | 3-6 | |

DOMAINES TECHNIQUES RECHERCHES (Int. Cl.4)

G 01 N

Le présent rapport a été établi pour toutes les revendications

| Lieu de la recherche | Date d'achèvement de la recherche | Examinateur |
|---|---|---|
| LA HAYE | 23-02-1989 | CARTAGENA Y ABELLA P. |

Office européen

des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | CHEMICAL ABSTRACTS, vol. 77, no. 15, 9 octobre 1972, page 84, résumé no. 97513v, Columbus, Ohio, US; E. KARLSSON et al.: "Purification of a presynaptic neurotoxin from the venom of the Australian tiger snake Notechis scutatus scutatus", & TOXICON 1972, 10(4), 405-13 <br> * Résumé * <br> --- | 3-6 | |
| A | BIOLOGICAL ABSTRACTS/RRM, vol. 33, 1987, Philadelphia, PA, US; S.R. LANGTON et al.: "A highly sensitive assay for serum phospholipase A2 (PLA2) using a monoclonal antibody", & ICSU, 6(0), no. 0, 1986, 108-9 <br> ----- | | |

DOMAINES TECHNIQUES RECHERCHES (Int. Cl.4)

Le présent rapport a été établi pour toutes les revendications

| Lieu de la recherche | Date d'achèvement de la recherche | Examinateur |
|---|---|---|
| LA HAYE | 23-02-1989 | CARTAGENA Y ABELLA P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)